# EUROPEAN PATENT APPLICATION

(11) **EP 4 122 431 A1**
(43) Date of publication of application: **25.01.2023**
(21) Application number: 20925317.8
(22) Date of filing: 24.12.2020
(51) Int. Cl.: A61F 5/34, A61F 13/00, A61B 17/135, A61H 9/00

(54) **COLD AND HOT COMPRESS PACK AND MANUFACTURING METHOD THEREFOR, AND COLD AND HOT COMPRESS CIRCULATION SYSTEM**

(30) Priority: 18.03.2020 CN 202010192947
(71) Applicant: Suzhou Microport Rehabtech (Group) Co., Ltd., Suzhou, Jiangsu 215000 (CN)
(72) Inventor: RUAN, Jiantao, Suzhou, Jiangsu 215000 (CN); DI, Pei, Suzhou, Jiangsu 215000 (CN); ZHAO, Jun, Suzhou, Jiangsu 215000 (CN)
(74) Representative: Lavoix
(86) International application number: PCT/CN2020/139176
(87) International publication number: WO 2021/184872

(57) **Abstract**

A cold and hot compress pack includes a first sheet (110), a second sheet (120), a third sheet (130), a first weld seam (210), a second weld seam (220), a gas port (310) and at least two liquid ports (320). The first sheet (110), the second sheet (120) and the third sheet (130) are sequentially stacked one above another. The first weld seam (210) joins the first sheet (110), the second sheet (120) and the third sheet (130) at a same planimetric position. The second weld seam (220) surrounds the first sheet (110), the second sheet (120) and the third sheet (130) and joins them together at a same planimetric position. The second weld seam (220) is joined to one end of the first weld seam (210), thereby forming a first cavity (141) between the first sheet (110) and the second sheet (120) and a second cavity (142) between the second sheet (120) and the third sheet (130). The gas port (310) is arranged between any two of the liquid ports (320) so as to be spaced apart from both. The at least two liquid ports (320) are in communication with the second cavity (142), and the gas port (310) is in communication with the first cavity (141). An end of the first weld seam (210) joined to the second weld seam (220) extends between the gas port (310) and any one of the liquid ports (320) while being spaced apart from both. Also disclosed are a corresponding manufacturing method and a cold and hot compress circulation system.

## Description

### TECHNICAL FIELD

The present invention relates to the technical field of medical apparatus and, in particular, to a cold and hot compress pack and a manufacturing method therefor, and a cold and hot compress circulation system.

### BACKGROUND

In the current medical practice, a cold and hot compress pack is often used to treat part of the body of a patient. However, the manufacturing of existing cold and hot compress packs is complicated, tedious and inefficient because it involves many steps in which multiple copper molds are used for welding.

### SUMMARY OF THE INVENTION

It is an object of the present invention to overcome the problem of complicated, tedious and inefficient manufacturing of conventional cold and hot compress packs by presenting a cold and hot compress pack, a manufacturing method therefor, and a cold and hot compress circulation system.

To this end, according to one aspect of the present invention, a cold and hot compress pack is provided, which comprises a first sheet, a second sheet, a third sheet, a first weld seam, a second weld seam, a gas port and at least two liquid ports,
the first sheet, the second sheet and the third sheet sequentially stacked one above another,
the first weld seam joining the first sheet, the second sheet and the third sheet at a same planimetric position,
the second weld seam surrounding the first sheet, the second sheet and the third sheet and joining them together at a same planimetric position, the second weld seam joined to one end of the first weld seam so that a first cavity is formed between the first sheet and the second sheet and a second cavity is formed between the second sheet and the third sheet,
the gas port arranged between any two of the liquid ports so as to be spaced apart from both, the at least two liquid ports in communication with the second cavity, the gas port in communication with the first cavity,
the first weld seam having an end that is joined to the second weld seam and extends between the gas port and any one of the liquid ports so as to be spaced apart from both.

Optionally, the cold and hot compress pack may further comprise a plurality of welding spots which are formed spaced apart from one another between the second sheet and the third sheet.

Optionally, the second weld seam may be corrugated in a direction in which the first sheet, the second sheet and the third sheet are stacked one above another.

Optionally, the cold and hot compress pack may further comprise a jacket arranged on a side of the first sheet away from the third sheet and/or on a side of the third sheet away from the first sheet.

Optionally, the cold and hot compress pack may further comprise a hook-and-loop fastener having hook and loop portions provided respectively to any two of the first sheet, the third sheet and the jacket.

Optionally, the jacket may be formed of a fabric coated with a waterproof coating or an antibacterial coating.

Optionally, the cold and hot compress pack may further a strap extending from the second weld seam in a plane.

Optionally, the jacket may be provided with a through hole allowing passage of the strap therethrough.

Optionally, the cold and hot compress pack may further a strap extending from the second weld seam in a plane.

Optionally, the strap may be made of a flexible non-stretchable material.

Optionally, the flexible non-stretchable material may include a woven material or a non-woven fabric.

Optionally, the first sheet, the second sheet and the third sheet may be made of flexible polymer material.

Optionally, the polymer material may include nylon or polyurethane.

According to another aspect of the present invention, there is also provided a method of making a cold and hot compress pack, which comprises:
arranging a gas port and at least two liquid ports so that the gas port is located between any two of the liquid ports and spaced apart therefrom;
welding a first sheet, a second sheet, and a third sheet at a same planimetric position to form a first weld seam; and
welding the first sheet, the second sheet and the third sheet at a same planimetric position, which surrounds all of the first sheet, the second sheet and the third sheet so as to form a second weld seam, forming a first cavity between the first sheet and the second sheet and a second cavity between the second sheet and the third sheet, and bringing the first cavity into communication with the gas port and bringing the second cavity into communication with the at least two liquid ports,
wherein the first weld seam and the second weld seam are configured so that an end of the second weld seam intersects with an end of the first weld seam and that the end of the first weld seam joined to the second weld seam extends between the gas port and any one of the liquid ports so as to be spaced apart from both.

Optionally, the method may further comprise, prior to the formation of the first weld seam, welding the second sheet and the third sheet to form a plurality of welding spots.

Optionally, the formation of the second weld seam may comprise welding the first sheet, the second sheet and the third sheet using a mold that is corrugated in a direction in which the first sheet, the second sheet and the third sheet are stacked so that the formed second weld seam is corrugated.

Optionally, the method may further comprise providing a jacket for the cold and hot compress pack so that the jacket is arranged on a side of the first sheet away from the third sheet and/or on a side of the third sheet away from the first sheet.

Optionally, the first weld seam and the second weld seam may be formed in a single pressing process using a single mold.

Optionally, the first weld seam and the second weld seam may be formed in a single process by ultrasonic welding with high-frequency ultrasonic acoustic vibrations.

According to yet another aspect of the present invention, there is also provided a cold and hot compress circulation system comprising the cold and hot compress pack as defined above, a connecting pipe and a cold and hot compress circulation device. The cold and hot compress pack is connected to the cold and hot compress circulation device by the connecting pipe configured for liquid and gas circulation.

In summary, the present invention provides a cold and hot compress pack, a manufacturing method therefor, and a cold and hot compress circulation system. The cold and hot compress pack includes a first sheet, a second sheet, a third sheet, a first weld seam, a second weld seam, a gas port and at least two liquid ports. The first sheet, the second sheet and the third sheet are sequentially stacked one above another. The first weld seam joins the first sheet, the second sheet and the third sheet at a same planimetric position. The second weld seam surrounds the first sheet, the second sheet and the third sheet and joins them together at a same planimetric position. The second weld seam is joined to one end of the first weld seam, thereby forming a first cavity between the first and second sheets and a second cavity between the second and third sheets. The gas port is arranged between any two of the liquid ports so as to be spaced apart from both. The at least two liquid ports are in communication with the second cavity, and the gas port is in communication with the first cavity. An end of the first weld seam joined to the second weld seam extends between the gas port and any one of the liquid ports while being spaced apart from both. With this arrangement, the first weld seam can be formed in a single welding process, resulting in the formation of the first and second cavities. In this way, the two liquid ports are isolated and a fluid path is formed. In addition, expansion of the first cavity is limited, and a simple fabrication process, increased productivity and lower fabrication cost are achieved.

### BRIEF DESCRIPTION OF THE DRAWINGS

Those of ordinary skill in the art would appreciate that the following drawings are presented merely to enable a better understanding of the present invention rather than to limit the scope thereof in any sense. In the drawings:
Fig. 1 is a schematic diagram showing a surface of a cold and hot compress pack according to an embodiment of the present invention;
Fig. 2 is a schematic cross-sectional view of the cold and hot compress pack according to an embodiment of the present invention;
Fig. 3 is a schematic side view of the cold and hot compress pack according to an embodiment of the present invention, showing a second weld seam;
Fig. 4 is a schematic diagram showing another surface of the cold and hot compress pack of Fig. 1; and
Fig. 5 is a schematic diagram of a cold and hot compress circulation device in communication with the cold and hot compress pack according to an embodiment of the present invention.

List of Reference Numerals:
110: First Sheet; 120: Second Sheet; 130: Third Sheet; 141: First Cavity; 142:
Second Cavity; 200: Welding Spots; 210: First Weld Seam; 220: Second Weld Seam; 300: Cold and hot compress circulation device; 310: Gas Port; 320: Liquid Ports; 400: Strap; 410: Hook-and-loop Fastener; 500: Connecting Pipe.

### DETAILED DESCRIPTION

Objects, features and advantages of the present invention will become more apparent upon reading the following more detailed description of the present invention, which is set forth by way of particular embodiments with reference to the accompanying drawings. Note that the figures are provided in a very simplified form not necessarily drawn to exact scale and for the only purpose of facilitating easy and clear despription of the embodiments. In addition, the structures shown in the figures are usually partially representations of their actual counterparts. In particular, as the figures would have different emphases, they are sometimes drawn to different scales.

As used herein, the singular forms "a", "an" and "the" include plural referents, unless the context clearly dictates otherwise. As used herein, the term "or" is generally employed in the sense of "and/or", and "several" of "at least one", unless the context clearly dictates otherwise.

The present invention seeks primarily to solve the problem of complicated, tedious and inefficient manufacturing of conventional cold and hot compress packs by providing a cold and hot compress pack, a manufacturing method therefor, and a cold and hot compress circulation system.

The following description is set forth with reference to the accompanying drawings.

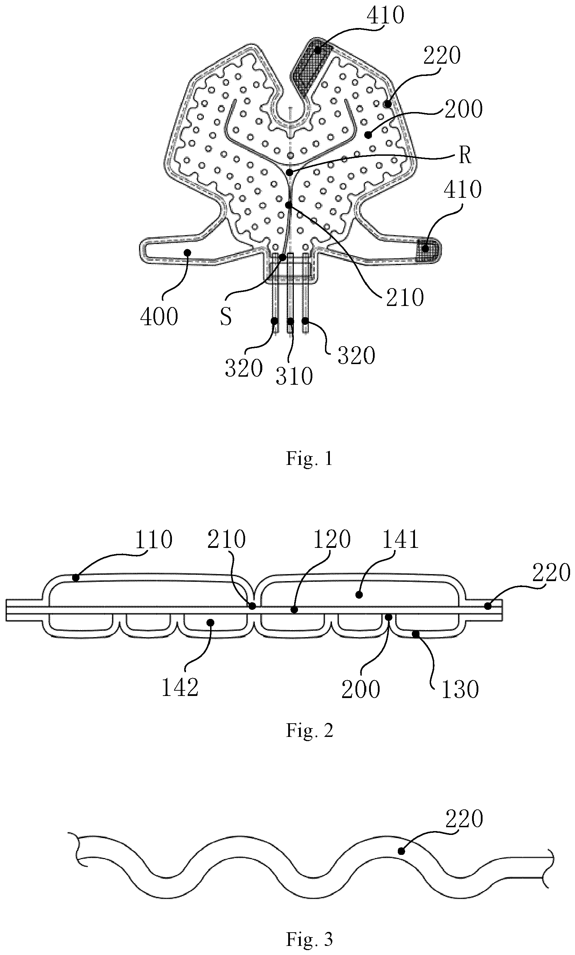

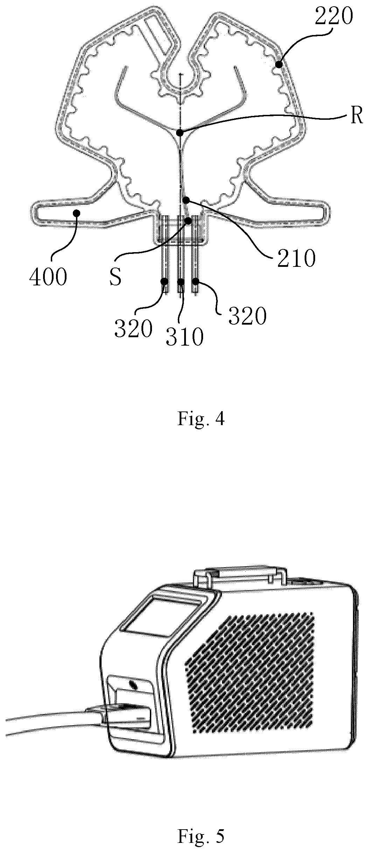

Reference will now be made to Figs. 1-5. Fig. 1 is a schematic diagram showing a surface of a cold and hot compress pack according to an embodiment of the present invention. Fig. 2 is a schematic cross-sectional view of the cold and hot compress pack according to an embodiment of the present invention. Fig. 3 is a schematic side view of the cold and hot compress pack according to an embodiment of the present invention, showing a second weld seam. Fig. 4 is a schematic diagram showing another surface of the cold and hot compress pack of Fig. 1. Fig. 5 is a schematic diagram of a cold and hot compress circulation device in communication with the cold and hot compress pack according to an embodiment of the present invention.

As shown in Figs. 1 and 2, in conjunction with Fig. 4, in an embodiment of the present invention, there is provided a cold and hot compress pack for providing a therapeutic effect to part of a patient's body by cold/hot compress. The cold and hot compress pack includes a first sheet 110, a second sheet 120, a third sheet 130, a first weld seam 210, a second weld seam 220, a gas port 310 and at least two liquid ports 320. In the example shown in Fig. 1, two liquid ports 320 are included, one of which is a liquid inlet port and the other is a liquid outlet port. The first sheet 110, the second sheet 120 and the third sheet 130 are stacked sequentially one above another. The first weld seam 210 joins the first sheet 110, the second sheet 120 and the third sheet 130 at a same planimetric position. The second weld seam 220 surrounds the first sheet 110, the second sheet 120 and the third sheet 130 and joins them together at a same planimetric position. The second weld seam 220 is joined to one end of the first weld seam. In this way, the first sheet 110 and the second sheet 120 define a first cavity 141 therebetween, and the second sheet 120 and the third sheet 130 define a second cavity 142 therebetween. The gas port 310 is arranged between any two of the liquid ports 320 so as to be spaced apart from both. The at least two liquid ports 320 are in communication with the second cavity 142, and the gas port 310 is in communication with the first cavity 141. The first weld seam 210 extends between the gas port 310 and any one of the liquid ports 320. In an exemplary embodiment, as shown in Figs. 1 and 4, with respect to a center line R of the cold and hot compress pack, the first weld seam 210 extends from its peripheral end S that is distant from the center line R along a curve that gradually approaches and then comes into coincidence with the center line R. This can result in a relatively balanced liquid distribution in the second cavity 142 on opposite sides of the first weld seam 210. The second weld seam 220 surrounds the first sheet 110, the second sheet 120 and the third sheet 130, and the second weld seam 220 is located in the same plane the three sheets are joined. That is, the second weld seam 220 can be formed by performing a single welding process on the three sheets. The first weld seam 210 is joined at the peripheral end S to the second weld seam 220. With this arrangement, both the first cavity 141 and the second cavity 142 are shaped to be curved by the first weld seam 210, with the two liquid ports 320 located respectively at starting and terminating ends of the curved second cavity 142. Of course, in other embodiments, more liquid ports 320 could be included, at least some of which are located at the starting end and the remaining ones are located at the terminating end of the curved shape.

In the cold and hot compress pack, a heat exchange liquid is usually circulated. Accordingly, it is necessary to provide a cavity for accommodating the circulation of the heat exchange liquid (the second cavity 142, in this embodiment). Moreover, during use, the second cavity 142 is oriented to face the part of the body being treated. Further, in this embodiment, the first cavity 141 is provided on the side of the second cavity 142 away from the body and serves as a pressurization cavity during use. The first cavity 141 can be inflated and expanded to exert a pressure via the second cavity 142 to the part of the body being treated, which results in a better fit between the second cavity 142 and the part of the body being treated and slows down the flow of the heat exchange liquid in the second cavity 142, thereby allowing its adequate heat exchange with the part of the body being treated. In addition, the pressure applied by the first cavity 141 to the part of the body being treated can reduce the occurrence of edema or the like. Conventionally, the first cavity 141 and the second cavity 142 were typically separately formed and then integrated into one piece using a pressing process. A cold and hot compress pack produced in this way requires the use of four sheets and multiple welding processes.

Further, conventionally, although one sheet could be omitted from the common sheets between the first cavity 141 and the second cavity 142 for the purpose of saving material, multiple welding processes were still necessary. Specifically, in some conventional designs, there was no weld seam arranged in the first cavity 141. This led to an isotropic gas pressure, which in turn often led to uncontrolled expansion and an undesirable fit of the second cavity 142. In order to mitigate uncontrolled expansion of the first cavity 141 and enable better pressure transfer therefrom to the second cavity 142, several weld seams A were usually arranged within the first cavity 141. Moreover, in order to slow down the flow of a heat exchange liquid within the second cavity 142 and thus enable adequate heat exchange between the heat exchange liquid and part of a patient's body being treated, several weld seams B were usually also provided to create a zigzag flow path consisting of several turns in the second cavity 142. In particular, since the first cavity 141 and the second cavity 142 shared a common sheet, after a certain location had been subject to a welding process for one cavity, it was difficult to perform another welding process for the other cavity at the same location. Therefore, it was often necessary to form the weld seams A in the first cavity 141 and the weld seams B in the second cavity 142 at different locations in a plane using two welding processes. As a consequence, the manufacturing may involve many steps and require the use of at least two molds (for A and B), which makes it expensive. In contrast to this, according to embodiments of the present invention, the weld seams A and B are brought into coincidence with each other to form the first weld seam 210. The first weld seam 210 can be formed by a single welding process using only one mold. In this way, the manufacturing can be accomplished in a simpler manner at lower cost with higher efficiency. Furthermore, the first weld seam 210 and the second weld seam 220 can be formed by a sing pressing process using the same mold and, for example, ultrasonic welding with high-frequency ultrasonic acoustic vibrations.

In order to enable the first weld seam 210 to create a flow path for the heat exchange liquid within the second cavity 142, which can avoid the heat exchange liquid from flowing out of the second cavity 142 immediately after it entered the second cavity 142 and thus leading to a thermal short circuit, an end of the first weld seam 210 where it is joined to the second weld seam 220 is configured to extend between the gas port 310 and any one of the liquid ports 320 while being spaced apart from both. With this arrangement, the two liquid ports 320 are located on opposite sides of the first weld seam 210, avoiding the occurrence of a thermal short circuit. In this way, the first weld seam 210 not only delimits different flow path portions for the two liquid ports 320 but also enables control expansion of the first cavity 141.

Preferably, the cold and hot compress pack further includes a plurality of welding spots 200 which are spaced apart from one another and formed between the second sheet 120 and the third sheet 130. If the second cavity 142 has a relative large fluid path width, when the pack is wrapped around the part of the body being treated, folding or twisting tends to occur, which may lead to an uneven distribution of the heat exchange liquid when it is flowing within the second cavity 142. Arranging the plurality of welding spots 200 can make the heat exchange liquid more evenly distributed within the second cavity 142, thus avoiding the problem of an uneven distribution of the heat exchange liquid caused by a shape change of the second cavity 142.

As shown in Fig. 3, preferably, the second weld seam 220 is corrugated in the stacking direction of the first sheet 110, the second sheet 120 and the third sheet 130. The inventors have found that existing cold and hot compress packs typically have flat edges. In practical use, when such a pack is wrapped around a part of a patient's body being treated, the edge of the pack may be folded or twisted, leading to a fluid path therein being blocked. Through corrugating the second weld seam 220, deformation of the pack when it is in a curved configuration can be effectively reduced, thus avoiding possible blockage or flexing in the configuration. Moreover, the corrugated shape can improve a user's comfort by avoiding the formation of one or more stiff portions, which may result from folding of a flat edge being used and may cause, when compressing the user, discomfort thereof. It would be appreciated that, here, by "corrugated", it is meant to refer to any of various shapes consisting of joined segments that are alternatively convex and concave in the stacking direction of the first sheet 110, the second sheet 120 and the third sheet 130 (referred to hereinafter as the "vertical direction", for each of description), such as a wavy shape consisting of curved or straight such segments, but not limited to any particular form in the direction of a plane in which the first sheet 110, the second sheet 120 and the third sheet 130 extend. In the example of Fig. 1, the second weld seam 220 is corrugated or wavy not only in the vertical direction but also in the direction of the plane.

Optionally, the cold and hot compress pack may further include a jacket (not shown) disposed on the side of the first sheet 110 away from the third sheet 130 and/or the side of the third sheet 130 away from the first sheet 110. In order to improve comfort when the cold and hot compress pack is fitted against the part of the body being treated, the jacket may be disposed on the side of the third sheet 130 away from the first sheet 110, or on the side of the first sheet 110 away from the third sheet 130. Of course, it may be disposed on both the sides. Further, in order to enable more reliable hygienic disposal of the cold and hot compress pack, the jacket is preferred to overall cover the first sheet 110, the second sheet 120 and the third sheet 130. With this arrangement, when the jacket is soiled or worn, it can be replaced without having to discard the entire cold and hot compress pack.

In some embodiments, the first sheet 110, the second sheet 120 and the third sheet 130 may be made of flexible polymer materials, such as nylon or polyurethane materials. The jacket may be made of a fabric, which may be coated with a waterproof coating, antibacterial coating or the like. The cold and hot compress pack further includes a strap 400 extending from the second weld seam 220 in a plane. Alternatively, the strap 400 may extend from an edge of the jacket in a plane. The strap 400 may be attached to any one of the first sheet 110, the second sheet 120 and the third sheet 130 so as to extend outward from the second weld seam 220, as shown in Fig. 1. In this way, the cold and hot compress pack can be reliably wrapped around the part of the body being treated. In this case, the jacket may be provided with a through hole allowing passage of the strap 400 therethrough. Of course, in other embodiments, the strap 400 may be secured to the jacket. The strap 400 is substantially formed of a flexible non-stretchable material, such as a woven or non-woven fabric. Those skilled in the art can choose proper materials for the various components of the cold and hot compress pack and appropriately configure them, as they conventionally do.

Optionally, the cold and hot compress pack may further include a hook-and-loop fastener 410 with hook and loop portions, which are disposed respectively on any two of the first sheet 110, the third sheet 130 and the jacket. Preferably, the hook-and-loop fastener 410 is disposed at one end of the strap 400 and adapted to secure the cold and hot compress pack. In the example of Fig. 1, the hook and loop portions of the hook-and-loop fastener 410 are disposed respectively on a front side of the first sheet 110, a back side of the third sheet 130 and the end of the strap 400. It is to be noted that, the cold and hot compress pack in the present embodiment is not limited to having the shape shown in Fig. 1, because depending on the part of the body to be treated, it may also have another shape, such as rectangular or elongated. According to the shape of the cold and hot compress pack, a person skilled in the art can properly determine the locations of the hook-and-loop fastener 410 and strap 400 or arrange multitudes of them.

In order to solve the above problem, embodiments of the present invention further provide a method for manufacturing a cold and hot compress pack, which includes the steps as follows:
Step S 1: Arranging a gas port 310 and at least two liquid ports 320 in such a manner that the gas port 310 is located between any two of the liquid ports 320 while being spaced apart therefrom.
Step S2: Welding a first sheet 110, a second sheet 120 and a third sheet 130 at a same planimetric position to form a first weld seam 210. Preferably, the first weld seam 210 extends from its peripheral end S that is distant from a center line R of the cold and hot compress pack along a curve that gradually approaches and then comes into coincidence with the center line R.
Step S3: Welding the first sheet 110, the second sheet 120 and the third sheet 130 at a same planimetric position, which surrounds all of the first sheet 110, the second sheet 120 and the third sheet 130, thereby forming a second weld seam 220. As a result, a first cavity 141 is formed between the first sheet 110 and the second sheet 120, and a second cavity 142 is formed between the second sheet 120 and the third sheet 130. The first cavity 141 is brought into communication with the gas port 310, and the second cavity 142 is brought into communication with all the liquid ports 320.

The first weld seam 210 and the second weld seam 220 are so formed that the second weld seam 220 is joined at one end thereof to the first weld seam 210. An end of the first weld seam 210 where it is joined to the second weld seam 220 extends between the gas port 310 and any one of the liquid ports 320 while being spaced apart from both.

It is to be noted that steps S2 and step S3 are not limited to being performed separately. In some embodiments, a single pressing process using a single mold is performed, i.e., steps S2 and step S3 are carried out simultaneously. In this way, the first weld seam 210 and the second weld seam 220 can be formed in the same process using ultrasonic welding with high-frequency ultrasonic acoustic vibrations.

Additionally, prior to the formation of the first weld seam 210, the method may further include welding the second sheet 120 and the third sheet 130 to form a plurality of Welding spots 200. The plurality of Welding spots 200 may be formed in case of a relatively large fluid path width of the second cavity 142. Preferably, the welding spots 200 are formed prior to the formation of the first weld seam 210.

Preferably, the formation of the second weld seam 220 may include welding the first sheet, the second sheet and the third sheet using a mold that is corrugated in a stacking direction of the first sheet 110, the second sheet 120 and the third sheet 130 so that the formed second weld seam 220 is also corrugated. The method may further include providing a jacket for the cold and hot compress pack. The jacket may be disposed on the side of the first sheet 110 away from the third sheet 130 and/or on the side of the third sheet 130 away from the first sheet 110. Reference can be made to the above description for details in the arrangements and structures of the second weld seam 220 and the jacket, and a duplicate description thereof will be omitted.

As shown in Fig. 5, embodiments of the present invention further provide a cold and hot compress circulation system, which includes the cold and hot compress pack as defined above and a cold and hot compress circulation device. The cold and hot compress pack is connected to the cold and hot compress circulation device 300 by a connecting pipe 500. Generally, the cold and hot compress circulation device 300 may supply a heat exchange liquid (e.g., cold or hot water) and gas (e.g., air). The connecting pipe 500 includes an inlet pipe, a return pipe and a gas pipe. The heat exchange liquid flows in and out of the second cavity 142 through the inlet pipe, the return pipe and the liquid ports 320 of the cold and hot compress pack, and the gas flows in and out of the first cavity 141 through the gas pipe and the gas port. Since the cold and hot compress circulation system includes the cold and hot compress pack as defined above, it also has the benefits of the cold and hot compress pack. Those skilled in the art can choose a suitable conventional product as the cold and hot compress circulation device. Other structural and mechanistic details of the cold and hot compress circulation device will be omitted herein.

In summary, the present invention provides a cold and hot compress pack, a manufacturing method therefor and a cold and hot compress circulation system. The cold and hot compress pack includes a first sheet, a second sheet, a third sheet, a first weld seam, a second weld seam, a gas port and at least two liquid ports. The first sheet, the second sheet and the third sheet are sequentially stacked one above another. The first weld seam joins the first sheet, the second sheet and the third sheet at a same planimetric position. The second weld seam surrounds the first sheet, the second sheet and the third sheet and joins them together at a same planimetric position. The second weld seam is joined to one end of the first weld seam. In this way, a first cavity is formed between the first and second sheets, and a second cavity between the second and third sheets. The gas port is arranged between any two of the liquid ports so as to be spaced apart from both. The at least two liquid ports are in communication with the second cavity, and the gas port is in communication with the first cavity. An end of the first weld seam where it is joined to the second weld seam extends between the gas port and any one of the liquid ports while being spaced apart from both, thereby isolating the two liquid ports and forming a fluid path. In addition, expansion of the first cavity is limited, and a simple manufacturing process, increased productivity and lower fabrication cost are achieved.

The description presented above is merely that of a few preferred embodiments of the present invention and is not intended to limit the scope thereof in any sense. Any and all changes and modifications made by those of ordinary skill in the art based on the above teachings fall within the scope as defined in the appended claims.

## Claims

1. A cold and hot compress pack, comprising a first sheet, a second sheet, a third sheet, a first weld seam, a second weld seam, a gas port and at least two liquid ports,
the first sheet, the second sheet and the third sheet sequentially stacked one above another,
the first weld seam joining the first sheet, the second sheet and the third sheet at a same planimetric position,
the second weld seam surrounding the first sheet, the second sheet and the third sheet and joining the first sheet, the second sheet and the third sheet together at a same planimetric position, the second weld seam joined to an end of the first weld seam, so that a first cavity is formed between the first sheet and the second sheet and a second cavity is formed between the second sheet and the third sheet,
the gas port arranged between any two of the liquid ports and spaced apart from both, the at least two liquid ports in communication with the second cavity, the gas port in communication with the first cavity,
the first weld seam having an end that is joined to the second weld seam and extends between the gas port and any one of the liquid ports so as to be spaced apart from both.

2. The cold and hot compress pack according to claim 1, further comprising a plurality of welding spots which are formed spaced apart from one another between the second sheet and the third sheet.

3. The cold and hot compress pack according to claim 1 or 2, wherein in a direction in which the first sheet, the second sheet and the third sheet are stacked one above another, the second weld seam is corrugated.

4. The cold and hot compress pack according to any one of claims 1 to 3, further comprising a jacket arranged on a side of the first sheet away from the third sheet and/or on a side of the third sheet away from the first sheet.

5. The cold and hot compress pack according to claim 4, further comprising a hook-and-loop fastener having a hook portion and a loop portion that are provided respectively to any two of the first sheet, the third sheet and the jacket.

6. The cold and hot compress pack according to claim 4 or 5, wherein the jacket is formed of a fabric coated with a waterproof coating or an antibacterial coating.

7. The cold and hot compress pack according to any one of claims 4 to 6, further comprising a strap extending from the second weld seam in a plane.

8. The cold and hot compress pack according to claim 7, wherein the jacket is provided with a through hole allowing passage of the strap therethrough.

9. The cold and hot compress pack according to any one of claims 1 to 3, further comprising a strap extending from the second weld seam in a plane.

10. The cold and hot compress pack according to any one of claims 4 to 6, further comprising a strap secured to the jacket.

11. The cold and hot compress pack according to any one of claims 7 to 10, wherein the strap is made of a flexible non-stretchable material.

12. The cold and hot compress pack according to claim 11, wherein the flexible non-stretchable material includes a woven material or a non-woven fabric.

13. The cold and hot compress pack according to any one of claims 1 to 12, wherein the first sheet, the second sheet and the third sheet are made of flexible polymer material.

14. The cold and hot compress pack according to claim 13, wherein the polymer material includes nylon or polyurethane.

15. A manufacturing method for a cold and hot compress pack, comprising:
arranging a gas port and at least two liquid ports so that the gas port is located between any two of the liquid ports and spaced apart therefrom;
welding a first sheet, a second sheet, and a third sheet at a same planimetric position to form a first weld seam; and
welding the first sheet, the second sheet and the third sheet at a same planimetric position, which surrounds all of the first sheet, the second sheet and the third sheet, so as to form a second weld seam, forming a first cavity between the first sheet and the second sheet and a second cavity between the second sheet and the third sheet, and bringing the first cavity into communication with the gas port and bringing the second cavity into communication with the at least two liquid ports,
wherein the first weld seam and the second weld seam are configured so that an end of the second weld seam intersects with an end of the first weld seam and that the end of the first weld seam joined to the second weld seam extends between the gas port and any one of the liquid ports so as to be spaced apart from both.

16. The manufacturing method for a cold and hot compress pack according to claim 15, further comprising, prior to the formation of the first weld seam, welding the second sheet and the third sheet to form a plurality of welding spots.

17. The manufacturing method for a cold and hot compress pack according to claim 15 or 16, wherein the formation of the second weld seam comprises welding the first sheet, the second sheet and the third sheet using a mold that is corrugated in a direction in which the first sheet, the second sheet and the third sheet are stacked so that the formed second weld seam is corrugated

18. The manufacturing method for a cold and hot compress pack according to any one of claims 15 to 17, further comprising providing a jacket for the cold and hot compress pack so that the jacket is arranged on a side of the first sheet away from the third sheet and/or on a side of the third sheet away from the first sheet.

19. The manufacturing method for a cold and hot compress pack according to any one of claims 15 to 18, wherein the first weld seam and the second weld seam are formed in a single pressing process using a single mold.

20. The manufacturing method for a cold and hot compress pack according to claim 19, wherein the first weld seam and the second weld seam are formed in a single process by ultrasonic welding with high-frequency ultrasonic acoustic vibrations.

21. A cold and hot compress circulation system, comprising the cold and hot compress pack according to any one of claims 1 to 14, a connecting pipe and a cold and hot compress circulation device, wherein the cold and hot compress pack is connected to the cold and hot compress circulation device by the connecting pipe configured for liquid and gas circulation.
